# EUROPEAN PATENT APPLICATION

(11) **EP 3 460 035 A1**
(43) Date of publication of application: **27.03.2019**
(21) Application number: 17192574.6
(22) Date of filing: 22.09.2017
(51) Int. Cl.: C12M 1/00, C12N 1/06

(54) **EXTRACTION OF METABOLITES FROM ORGANISMS CAPABLE OF PHOTOSYNTHESIS**

(71) Applicant: P3 B.V., 7861 TB Oosterhesselen (NL)
(72) Inventor: HULSHOFF, Hendrik Jan, 8701 DV BOLSWARD (NL); JAGER, Filips Gustaaf Hendrik, 7861 TB Oosterhesselen (NL); VAN VELZEN, Dick, 2343 BR OEGSTGEEST (NL)
(74) Representative: van Dam, Vincent

(57) **Abstract**

The present invention relates to an assembly for use in a process of treatment of a suspension containing organisms, capable of photosynthesis and containing metabolites and/or parts of said organisms. The invention also relates to a method for treatment of a suspension containing organisms, capable of photosynthesis and containing metabolites and/or parts of said organisms. The invention in particular aims at the facilitation of extraction of valuable metabolites from said organisms and/or said parts thereof.

## Description

The present invention relates to an assembly for use in a process of treatment of a suspension containing organisms, capable of photosynthesis and containing metabolites and/or parts of said organisms. The invention also relates to a method for treatment of a suspension containing organisms, capable of photosynthesis and containing metabolites and/or parts of said organisms. The invention in particular aims at the facilitation of extraction of valuable metabolites from said organisms and/or said parts thereof.

### BACKGROUND

Organisms capable of photosynthesis convert light energy into chemical energy by converting carbon dioxide and water into the end-products oxygen and carbohydrates.

Algae form an example of such organisms, and are well-known as efficient producers of biomass. During photosynthesis, algae utilize carbon dioxide and light in the presence of water to produce oxygen and biomass. The algae produce metabolites such as lipids, vegetable oils and proteins which can be used for various purposes, for example as a source for human or animal nutrition or biofuel.

In order to extract these metabolites from the algae various methods are available.

A number of methods use electrical treatment of the algae to disrupt algae cells to achieve the release of intracellular matter. Such treatment is also known as electroporation. Examples of such methods are described in US2016/298104 (A1) and US2012/0021481 A1.

The inventors have observed that electroporation methods as currently applied in the art often do not lead to complete disruption of algal cells and that rather high voltages and consequently high energy input is needed to release the intracellular content of the algae. As a consequence further treatment of electroporated cells is needed, which involves additional work, equipment and energy.

### SUMMARY OF THE INVENTION

With the present invention the inventors provide an assembly and a method that facilitate the extraction of metabolites. Accordingly, the invention opens the possibility to extract metabolites from organisms capable of photosynthesis, such as algae or phytoplankton, while requiring only a low energy input and a minimal amount of equipment and work, and enabling easy purification after lysis of the cells.

In a first aspect the invention relates to an assembly for use in treatment of a suspension containing organisms, capable of photosynthesis and containing metabolites, and/or parts of said organisms, said assembly comprising a tube, wherein said tube comprises a tapered portion; and a rotatable conical element which is connected to a rotatable shaft capable of driving rotation of the conical element in the rotation direction of the rotatable shaft, wherein the rotatable element is positioned in the tube such that at least a portion of it defines a cleft between its outer surface and the inner surface of at least a portion of the tapered portion of the tube, which cleft has dimensions that allow grinding and fractionation of material present in said suspension in said cleft when said conical element rotates; and wherein electrodes are comprised in the rotatable conical element and the tapered portion which are configured to apply a pulsed electric field to the suspension in the cleft.

In a second aspect the invention relates to a method for treatment of a suspension containing organisms, capable of photosynthesis and containing metabolites, or parts of said organisms, said method comprising: passing said suspension through the cleft of the assembly defined in the first aspect of the invention; applying a pulsed electric field to the suspension in the cleft; rotating said conical element to grind and fractionate material present in said suspension in said cleft; passing the suspension containing the ground and fractionated material out of the cleft.

### DESCRIPTION OF THE DRAWINGS

Fig.1A shows a schematic representation of a cross-section of a first exemplary embodiment of the assembly of the invention.
Fig.1B shows a schematic view of the conical element and drive shaft of which a cross-section is shown in Fig.1A.
Fig.2A shows a schematic representation of a cross-section of a second exemplary embodiment of the assembly of the invention.
Fig. 2B shows a schematic view of the conical element and drive shaft of which a cross-section is shown in Fig.2A.

### DETAILED DESCRIPTION

The invention is based on the principle of combining electroporation with mechanical grinding and fractionation of organisms capable of photosynthesis and or parts thereof in a suspension in order to facilitate and improve the extraction of valuable metabolites by means of a conical grinding element with an integrated electrode in a tube which also has an integrated electrode to allow the application of an pulsed electric field.

In accordance with the invention organisms, capable of photosynthesis and containing metabolites, and/or parts of these organisms are treated by passing a suspension of said organisms and/or parts thereof through the assembly of the invention; applying a pulsed electric field to the suspension; rotating the conical element of the assembly of the invention to grind and fractionate said organisms or parts thereof; and passing the suspension containing the ground and fractionated material out of the assembly for further treatment, such as further extraction and/or purification of the metabolites contained in the organisms.

Metabolites of interest may be selected from the group of proteins, peptides, carbohydrates, lipids, omega 3 fatty acids, trace elements, vitamins, minerals, beta carotene and pigments or combinations thereof.

The organisms used for purposes of the invention are organisms capable of photosynthesis and which contain valuable metabolites. Suitable organisms for purposes of the invention are phytoplankton or algae. Examples of such organisms include algal cells selected from a division comprising *Chlorophyta* (for example *Dunaliella* sp.), *Cyanophyta* (Cyanobacteria), *Rhodophyta* (red algae), and *Heterokontophyta.*

It is preferred to use microalgae, because these are an attractive food and food supplement source. Microalgae are rich in valuable metabolites. Microalgae may be selected from a class comprising Bacillariophyceae, Eustigmatophyceae, and Chrysophyceae, for instance from the microalgal genera selected from the group consisting of *Nannochloropsis, Chlorella, Dunaliella, Scenedesmus, Selenastrum, Oscillatoria, Phormidium, Spirulina, Amphora,* and *Ochromonas.* A suitable organism for purposes of the invention is *Chlorella vulgaris.*

The organisms are preferably produced in a bioreactor. In such a bioreactor the organisms are grown to a density which allows downstream extraction of metabolites in an economic way. In order to allow extraction of intracellular metabolites from organisms grown in a bioreactor, in accordance with the invention organisms are harvested and passed to the assembly according to the invention. Optionally the organisms are concentrated before downstream treatment. Suspensions can for instance be introduced into the assembly of the invention that are concentrated up to 50-60% vol/vol of algae, such as 20-30 % vol/vol of algae.

It is preferred that the assembly is configured in line with a bioreactor. Suitable tube and valve configurations can be used to connect the assembly according to the invention with a bioreactor. The inline arrangement of the assembly of the invention with a bioreactor enables sterile and continuous harvesting and downstream treatment of the organisms.

After harvest, optional concentration and any other optional further pre-treatment the suspension containing the organisms can be passed into the assembly of the invention. In the assembly of the invention the suspension is subjected to a a pulsed electric field combined by mechanical grinding and fractionation of the material present in the suspension by the action of the rotating conical element. The combination of electroporation and mechanical grinding has the advantage that cells or organelles can be fractionated in one action. The invention therefore enables the possibility to treat organisms by electrophoresis and mechanical grinding in one and the same device. Further, only low electrical field strengths are necessary to obtain high levels of rupture of the cells or organelles inside the cells by mechanical grinding.

The pulsed electrical field is effected by the electrodes that are provided in the rotatable conical element and the tapered portion of the tube wherein the conical element is positioned. The electrodes are configured to apply a pulsed electric field to the suspension in the cleft. Because of the small dimensions of the cleft, the energy of the pulsed electric fields enters the intracellular matter of the cells or cell material in the suspension efficiently, resulting in optimal disruption and fractionation of cells and cell material.

Because of the rotating action of the conical element and to provide optimal pulsed electric fields it is preferred that the electrodes are in the form of first and second rings, wherein the first ring is circumferentially embedded in the inner surface of the tapered portion and the second ring is circumferentially embedded in the outer surface of the rotatable conical element.

It is preferred that electrodes in the rotatable conical element and the tapered portion are positioned nearby the point of entry of the suspension in the cleft, for instance at the point of entry of the suspension in the cleft. This way the membrane structures of the organisms in the suspension are disrupted at entry of the suspension into the cleft so that the level of mechanical fractionation by the grinding action of the conical element is enhanced and facilitated. For efficiency's sake, it is preferred that the electrodes are positioned diametrically opposed to each other.

The electrodes are configured to produce high voltage pulses to the suspension flowing between them. The pulses may typically have a width in the range of 1 to 100 µsec and a pulse frequency (i.e. pulse rate) in the range of 1 to 10,000 pulses/sec. The electrodes can be configured to produce high voltage pulses with an electric field intensity up to 80 kV/cm. Because of the combined action of grinding and electroporation low electric field intensities may suffice to provide a fully disrupted and fractionated suspension. This saves energy. For instance, suitable electric field intensities may be lower than 1 kV/cm, such as between 0,1 and 0,95 kV/cm.

The pulsed electric fields (PEFs) may be generated by any conventional PEF generating system. Such systems generally include a high voltage generator, a pulse generating circuit (e.g., an array of capacitors and inductors, and switching devices) that produces high voltage pulses, and electrodes which in case of the present invention are located in the tapered section of the tube and in the rotatable conical element. When the electrodes receive the high voltage pulses, they produce an electric field to which the suspension is exposed. For this purpose electric wiring may be provided inside the drive shaft of the conical element and on the outside and through the wall of the tube. The rotatable shaft to which the conical element is connected is typically hollow, and may comprise wiring to connect the electrode to the PEF generating system. To enable rotation of the conical element electricity can be transmitted between the electrode of the conical element and the wiring in a wireless way, for instance by means of any suitable wireless power coupling. Any other suitable configuration that allows rotation of the conical element and generation of a pulsed electric field between the electrodes is also possible and can be realised according to techniques known in the art.

It is possible to subject the suspension with organisms to a pre-treatment before electroporation and grinding. In a preferred embodiment the method of the invention therefore comprises a step of sonicating the suspension before the suspension enters the cleft of the assembly of the invention. This sonication step disrupts the cells into chunks of cells. Typically ultrasonic frequencies are used for this purpose (> 20 kHz). By using the sonication pre-treatment, in the subsequent electroporation step the energy of the pulsed electric fields directly enters the intracellular matter of the cells instead of being absorbed by the cell wall. This way the pulsed electric field can disrupt intracellular structures that have a bilayer membrane. This way in particular the smooth endoplasmic reticulum (SER) and the rough endoplasmic reticulum (RER) are disrupted. This is an important advantage because it is in particular these organelles that contain valuable oils and lipids. Following disruption of these organelles by the pulsed electric fields, these organelles are then further fractionated by the grinding action of the rotating conical element, after which further extraction and purification of the desired compounds may follow. The provision of a sonication pre-treatment step before the steps of electroporation and grinding allows the application of low electric field intensities, for instance lower than 1 kV/cm, such as between 0,1 and 0,95 kV/cm, whilst still achieving proper disruption of intracellular bilayer structures such as the SER and RER. In order to realize the sonication pre-treatment it is preferred that a sonicator element is positioned on the tip of the conical element of the assembly of the invention, wherein the sonicator element is configured to transfer ultrasonic energy from the sonicator element to the suspension before its entry into the cleft. This embodiment therefore enables the possibility to treat organisms by sonication, electrophoresis and mechanical grinding with one and the same device.

In order to obtain proper grinding and fractionation of cells or organelles in the suspension, the cleft of the assembly of the invention has dimensions that allow grinding and fractionation of material present in said suspension in said cleft when said conical element rotates. In this respect it is preferred that the width of the cleft is 5 µm or less. Suitable widths may be between 2 and 5 µm. Cleft width may be adjusted by means of adjusting the position of the drive shaft.

In another embodiment the tube is oriented vertically and the rotatable conical element is configured in the tube such that the element is capable to move freely to at least a predetermined extent in the vertical direction so that the rotatable conical element is effectively supported by the tapered portion of the tube. In order to achieve a certain degree of free movement in vertical direction either the conical element may be mounted on the drive shaft in a way that allows a degree of free vertical movement of the conical element or the drive shaft may be mounted to its drive mechanism in a way that allows a degree of free vertical movement of the combination of the shaft and the conical element. Due to this extent of free vertical movement the conical element is in effect supported by the tapered section. This results in a very narrow cleft, which increase the effectivity of grinding, in particular in case of small sized material. Accordingly, this embodiment is in particular preferred in case a sonication pre-treatment is used, because effective grinding of relatively small intracellular organelles such as SER and RER requires a small cleft width. Because rotation of the conical element in this embodiment is accompanied with grinding of the surfaces of the conical element and the tapered section in contact with the conical element, it is preferred that at least the rotatable conical element and the tapered portion are made of the same hard material, such as glass. It is therefore preferred that at least the rotatable conical element and the tapered portion are made of glass material. For the same reason it is also preferred that the electrodes are covered with a glass layer.

It is to be understood that the term "conical element" encompasses any element comprising a conical portion suitable to provide a grinding action in combination with the tapered portion of the tube. The conical element may therefore have a truncated conical or frustoconical shape. In order to connect the electrode and optional sonicator element of the conical element to a PEF generating system or sonicator respectively the conical element may be equipped with wiring and/or other electronic equipment, such as wireless couplings etc. on its inside in any suitable way as long as the conical element can perform its rotating and grinding action. The conical element may thus be hollow to a suitable extent.

After grinding and fractionation, the material in the suspension may be subjected to further steps of extraction and/or purification of the metabolites from the ground and fractionated material. Such steps may involve the use of monophasic or biphasic solvent systems, filtration, drying etc. Further extraction and/or purification can be performed by means of methods known in the art.

### DETAILED DESCRIPTION OF THE DRAWINGS

The invention will now be further elucidated in the attached drawings. The following explanation is meant to illustrate and explain the invention and not to limit the claims.

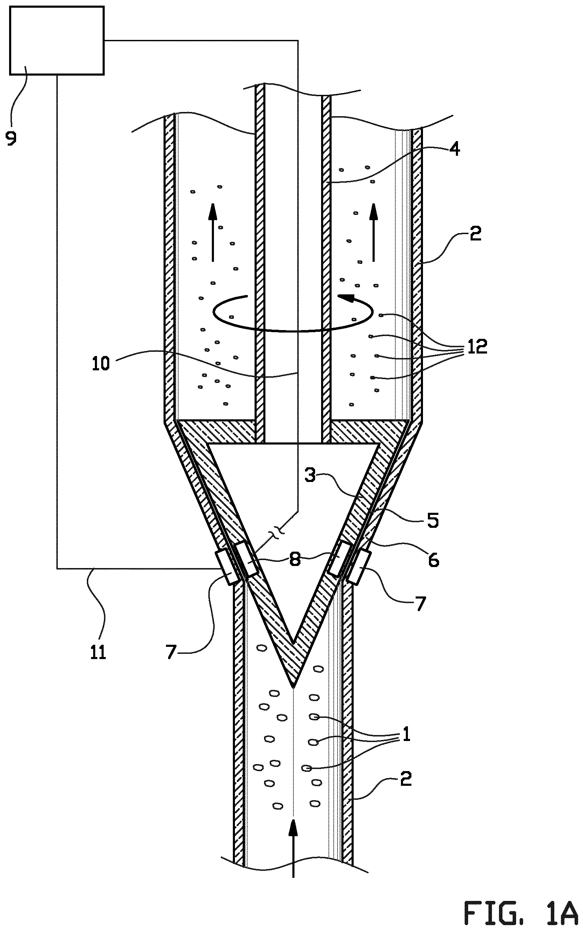

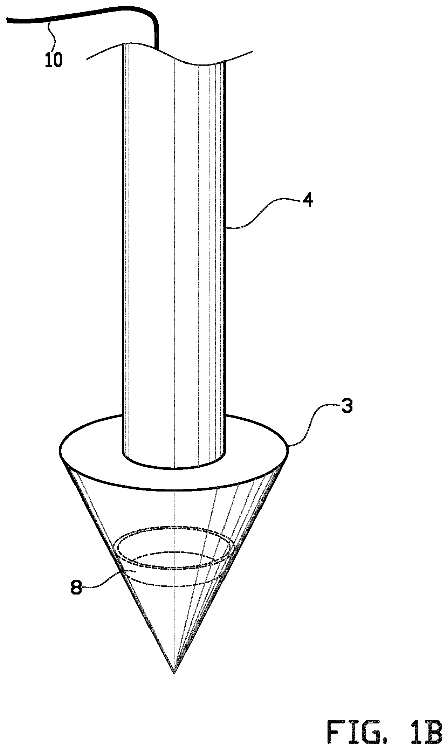

Fig. 1A shows a cross section of an exemplary assembly of the invention. When used for the post-harvest treatment of algae grown in a bioreactor, algae 1 enter a glass tube 2 of the assembly in the direction of the lower arrow. A rotatable conical element 3, made of glass and connected to a rotatable shaft 4 which drives rotation of the conical element in the rotation direction of the rotatable shaft is positioned in tube 2 such that a portion of it defines a cleft 5 between its outer surface and the inner surface of tapered portion 6 of the tube. A three dimensional view of this rotatable conical element 3 in connection to rotatable shaft 4 is shown in Fig. 1B. Electrodes 7, 8 are in the form of rings and are circumferentially embedded in the inner surface of the tapered portion (electrode 7) and in the outer surface of the rotatable conical element electrode 8). The electrodes 7,8 are covered with glass. The electrodes 7, 8 in the rotatable conical element 3 and the tapered portion 6 are positioned at the point of entry of the suspension in the cleft 5. The electrodes 7, 8 are configured to produce high voltage pulses to the suspension flowing between them. For this purpose they are connected to a PEF generating system 9 via electrical connections 10, 11. Part of connection 10 can be configured inside shaft 4. Connection 10 should be configured such that conical element 3 can rotate freely. Thus, upon entry into cleft 5 the algae are electroporated, resulting in rupture of the algae. This way the membrane structures of the organisms in the suspension are disrupted at entry of the suspension into the cleft. This results in more effective mechanical fractionation by the grinding action of the conical element 3. Cleft 5 has dimensions that allow grinding and fractionation of the disrupted algal material in the said suspension in said cleft when conical element 3 rotates, for instance in the direction of the circular arrow. In this exemplary embodiment it is preferred that these dimensions are realized by adjusting the position of the drive shaft 4 to obtain a cleft width of suitable dimensions, in this case suitably between 2 and 5 µm. After exit of the ground and fractionated material 12 from the cleft in the direction of the upper arrows, the suspension is passed to means (not shown) for further extraction and purification of metabolites from the ground and fractionated material.

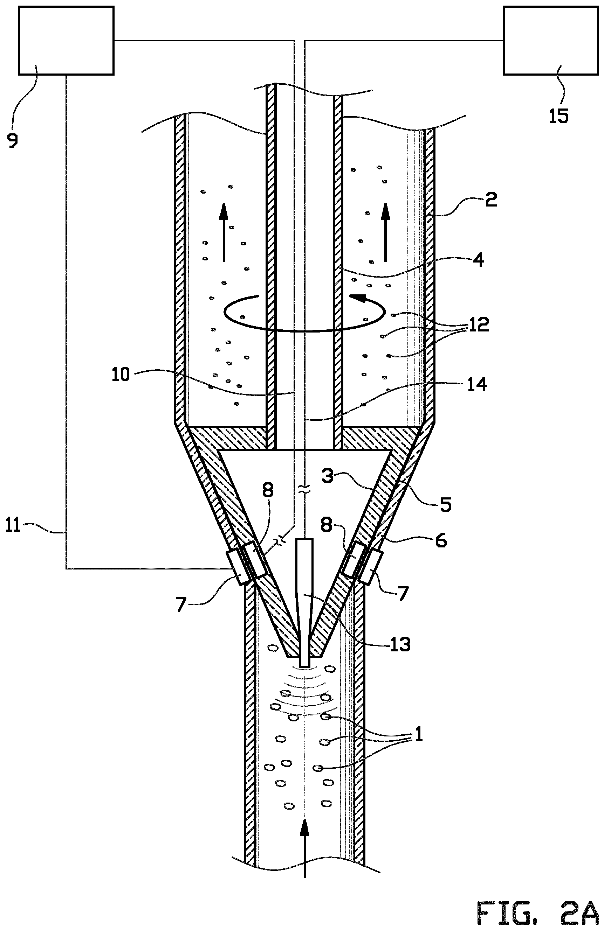

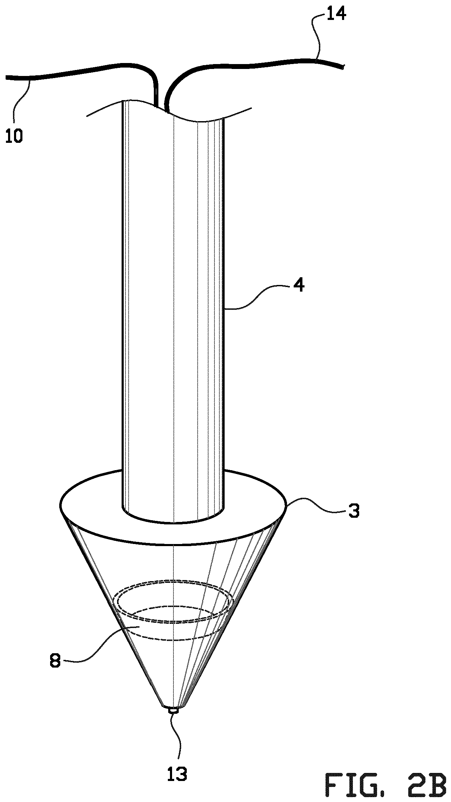

Fig. 2A shows a cross section of another exemplary assembly of the invention. Fig. 2B shows a three dimensional view of the rotatable conical element 3 in connection to rotatable shaft 4 as shown in Fig. 2A. The numbers in Fig. 2A and B have the same meaning as the numbers in Fig.1A and B and the elements represented by these numbers have the same function. Also in the embodiment shown in Fig. 2A and B, algae 1 grown in a bioreactor enter tube 2 of the assembly in the direction of the lower arrow. A rotatable conical element 3, connected to a rotatable shaft 4 which drives rotation of the conical element in the rotation direction of the rotatable shaft is positioned in tube 2 such that a portion of it defines a cleft 5 between its outer surface and the inner surface of tapered portion 6 of the tube. With the embodiment of the assembly of the invention shown in Fig. 2A and B it is possible to perform a sonication pre-treatment to the algae before they enter the cleft 5. For this purpose a sonicator element 13 is positioned on the tip of the conical element 3 of the assembly of the invention, wherein the sonicator element 12 is configured to transfer ultrasonic energy from the sonicator element to the suspension before its entry into the cleft. For this purposes the sonicator element 13 is connected via electric connection 14 with a sonicator device 15. Connection 14 should be configured such that rotation of conical element 3 is not hindered. This embodiment enables the possibility to treat algae by sonication, electrophoresis and mechanical grinding with one and the same device. Apart from the gain of efficiency by using one and the same device for this, it is also very energy efficient to extract metabolites of algae this way. By using the sonication pre-treatment, in the subsequent electroporation step by means of electrodes 7, 8 the energy of the pulsed electric fields directly enters the intracellular matter of the cells instead of being absorbed by the cell wall. This way the pulsed electric field can disrupt intracellular structures such as the smooth endoplasmic reticulum (SER) and the rough endoplasmic reticulum (RER) that contain valuable oils and lipids. Following disruption of these organelles by the pulsed electric fields, these organelles are then further fractionated by the grinding action of the rotating conical element 3, after which further extraction and purification of the desired compounds may follow. In this embodiment it is preferred that the tube 2 is oriented vertically and that rotatable conical element 3 is configured in the tube such that the element 3 is capable to move freely to at least a predetermined extent in the vertical direction so that the rotatable conical element 3 is supported by the tapered portion 6 of the tube 2. Due to this extent of free vertical movement the conical element 3 is in effect supported by the tapered section 6. This results in a very narrow cleft 5, which increases the effectivity of grinding, in particular in case of small sized material such as SER and RER. After exit of the ground and fractionated material 12 from the cleft 5 in the direction of the upper arrows, the suspension is passed to means (not shown) for further extraction and purification of metabolites from the ground and fractionated material.

## Claims

1. Assembly for use in treatment of a suspension containing organisms, capable of photosynthesis and containing metabolites, and/or parts of said organisms, said assembly
comprising a tube, wherein said tube comprises a tapered portion; and
a rotatable conical element which is connected to a rotatable shaft capable of driving rotation of the conical element in the rotation direction of the rotatable shaft, wherein the rotatable element is positioned in the tube such that at least a portion of it defines a cleft between its outer surface and the inner surface of at least a portion of the tapered portion of the tube, which cleft has dimensions that allow grinding and fractionation of material present in said suspension in said cleft when said conical element rotates; and
wherein electrodes are comprised in the rotatable conical element and the tapered portion which are configured to apply a pulsed electric field to the suspension in the cleft.

2. Assembly according to claim 1, wherein the width of the cleft is 5 µm or less.

3. Assembly according claim 1 or 2, wherein the tube is oriented vertically and wherein said rotatable conical element is configured in said tube such that the element is capable to move freely to at least a predetermined extent in the vertical direction so that the rotatable conical element is supported by the tapered portion of the tube.

4. Assembly according to any of the claims 1 to 3, wherein a sonicator element is positioned on the tip of the conical element, wherein the sonicator element is configured to transfer sonic energy from the sonicator element to the suspension before its entry into the cleft.

5. Assembly according to any of the claims 1 to 4, wherein at least the rotatable conical element and the tapered portion are of glass material.

6. Assembly according to any of the claims 1 to 5, wherein the electrodes are covered with a glass layer.

7. Assembly according to any of the claims 1 to 6, wherein the electrodes are in the form of first and second rings, wherein the first ring is circumferentially embedded in the inner surface of the tapered portion and the second ring is circumferentially embedded in the outer surface of the rotatable conical element.

8. Assembly according to any of the claims 1 to 7, wherein the electrodes in the rotatable conical element and the tapered portion are positioned nearby the point of entry of the suspension in the cleft.

9. Method for treatment of a suspension containing organisms, capable of photosynthesis and containing metabolites, and/or parts of said organisms,
said method comprising:
passing said suspension through the cleft of the assembly defined in any of the claims 1 to 8;
applying a pulsed electric field to the suspension in the cleft;
rotating said conical element to grind and fractionate material present in said suspension in said cleft;
passing the suspension containing the ground and fractionated material out of the cleft.

10. Method according to claim 9, further comprising a step of sonicating the suspension before said suspension enters the cleft.

11. Method according to claim 9 or 10, comprising
further extraction and/or purification of said metabolites from the ground and fractionated material.

12. Method according to any of the claims 9 to 11, wherein the metabolites are one or more selected from the group consisting of proteins, peptides, carbohydrates, lipids, omega 3 fatty acids, trace elements, vitamins, minerals, beta carotene and pigments or combinations thereof.

13. Method according to any of the claims 9 to 12, wherein the organisms capable of photosynthesis are phytoplankton.

14. Method according to claims 13, wherein the organisms capable of photosynthesis are microalgae.
